# EUROPEAN PATENT SPECIFICATION

(11) **EP 1 637 152 B1**
(45) Date of publication and mention of the grant of the patent: **10.08.2011**
(21) Application number: 04729596.9
(22) Date of filing: 27.04.2004
(51) Int. Cl.: A61K 36/258

(54) **A NOTOGINSENG SAPONIN INTRAVENOUS INJECTION AND THE METHOD FOR PREPARING THIS INJECTION**
INTRAVENÖSE NOTOGINSENG-SAPONIN-INJEKTION UND VERFAHREN ZUR VORBEREITUNG DIESER INJEKTION
INJECTION INTRAVEINEUSE DE SAPONINE DE NOTOGINSENG ET SON PROCEDE DE PRODUCTION

(30) Priority: 07.05.2003 CN 03130709
(43) Date of publication of application: 22.03.2006
(73) Proprietor: Li Min Pharmaceutical Factory of Livzon Pharmceutical Group, Shaoguan Guangdong 512028 (CN)
(72) Inventor: XIE, Weihong, Guandong Province 512028 (CN); LIU, Xuehua, Guangdong Province 512028 (CN); CHEN, Maoru, Guandong Province 512028 (CN)
(74) Representative: Ebner von Eschenbach, Jennifer
(86) International application number: PCT/CN2004/000409
(87) International publication number: WO 2004/098623

(56) References cited:
- CN-A- 1 157 720
- CN-A- 1 273 114

## Description

### Field of the Invention

The present invention relates to an intravenous injection and the preparation thereof, especially to the preparation of an intravenous injection of the saponins family of *Radix Notoginseng*.

### Background of the Invention

In Chinese traditional medicine, *Radix notoginseng* is the dry root of *Panax notoginseng* (Burk.) (F.H.Chen). The plant is harvested, washed, and dried before flowering in the fall. The roots are collected and sorted into root stalks, rootlets, and stem bases. The medicine is mild, has minimal odor and bitter-sweet taste.

The main chemical component of the *Radix notoginseng* extract is saponin. It is very effective for treatment of cardiac and cerebral vascular diseases. The *Radix notoginshen* preparations in today's markets are widely used in clinical practice, for example, Xueshuantong injection and Xuesaitong injection. It invigorates the circulation of blood, removes vascular stases, dilates blood vessels, and improves circulation. However, reactions of fluid infusions constantly occurs in clinical practices because of improper buffer, over prescription of medicines, existence of foreign particles, and improper compatibility of medicines, among others. Those reactions could bring severe pain to the patient and even be life threatening.

The research shows that foreign particles could cause pyrogen reaction, allergies, and so on when injected *in vivo.* Pharmacopoeia of many countries have strict requirements on the number of particles in large dose injections. However, there is no requirement for small dose injections and powder injections. Application of multiple medications is commonplace in today's intravenous injections. Research has shown increases in particle numbers in blood vessels after injection, especially when liquid and powder intravenous injections of Chinese traditional medicine are involved. Significant increases of insoluble particles have been observed when many of the Chinese traditional medicine injections are applied with fluid infusion. The main reason lies in the complexity of the Chinese traditional medicine components. Also, different medicinal manufacturing industries have different preparation methods, including the purification and extraction of the effective components. Some components in the medicinal solution, such as pigment, tannin, starch, and protein, exists in colloidal state. Many reactions occur after the medicinal solution is applied with the fluid infusion, including oxidation, polymerization, and precipitation of large amounts of insoluble particles from saponin and alkaloid due to changes in pH. A dilution buffer of 5% or 10% glucose injection, rather than physiological saline, is recommended when applying intravenous injections using Xuesaitong, red rooted salvia compound, acanthopanax, β-aescin sodium and other Chinese traditional medicines. This is because of high occurrence of large amount of insoluble particles from precipitating out if using physiological saline with the large array components of the Chinese traditional medicine extract. This could increase the possibility of fluid infusion reaction and cause medical incidences (Honglan Zhong, Analysis and solutions for fluid infusion reactions, Guangdong Medicine, 2002, vol. 12, number 4).

Improper compatibility of medicines could affect clarity of the compound solution, cause crystallization, and change pH (Xiujing Luo, 1999, The cause of fluid infusion fever, the preventive measures and thereof, Chinese Nursing Magazine, 34(10)). Reaction of fluid infusion is caused by over-dosage of multiple medicines and improper compatibility. The chances of contamination greatly increase because of repetitive injection of medicine into the medicine bottle in which multiple medicines are added. The clarity of the solution violates standard rules after adding 10% glucose injection with a chloromycetin/Vitamin C mixture, erythromycin/Vitamin C mixture, and tetracycline with other compounds. The solution then contains crystals, as well as pigmented and white clumps, among others. The pH of the 10% glucose solution for injection is decreased below the standard value after tetracycline is added. Medicinal particles could be generated if the powder injection is not dissolved thoroughly. The effective phase of penicillin G in glucose solution is only 2 hours. If it sits too long, the penicillin solution could even generate allergens which increase the chances of allergic reaction (Guitian Niu, Research and Experiments of Contamination in Clinic Fluid Infusion, Practical Nuring Magazine, 1993, 9(4), 25-27). On the other hand, chances of contamination greatly increase if any of the diluted solution sits for too long. The solubility, pH, age, and temperature could affect the solution after medicine homogenization and cause quality deterioration. This is especially true when there are large doses and multiple medicinal components involved because the contained pyrogen could accumulate to toxic levels, which will cause pyrogen reaction when injected *in vivo.*

The saponin could be hydrolyzed in solution with improper pH. The solubility could then decrease and white particles, clumps, and cotton-like solids could appear in the solution. The quality of the medicine solution could then be compromised. Thus it is very important to choose and maintain an appropriate pH in production and storage of the saponins of *Radix notoginshen* injection.

There are not only oral preparations, but also injections (small needle) used in today's clinics for the Chinese traditional medicine *Radix notoginseng*. The advantages of injection are efficiency and high biological utilization. The injection is especially beneficial to those patients who could not be treated with oral medicines and those who have serious conditions. However, small amounts of injection solution could be contaminated in practice. The operating procedures are complicated, which could add a greater burden to the medical practitioners, and cause the patients to undergo fluid infusion reactions.

### Summary of the Invention

The present invention provides a method for preparing a composition for intravenous injection comprising *Radix Panax Notoginseng* saponins wherein the method is characterized by the steps of
(1) diluting iso-osmotic solution comprising sodium chloride, glucose or sorbitol with distilled water to a concentration of 80 - 300 mg/ml of said sodium chloride, glucose or sorbitol, and filtering the same through active carbon;
(2) stirring Radix Panax Notoginseng saponins and dissolving in the filtrate;
(3) adding pH stabilizer to the filtrate and adjusting the pH value to 6, wherein the pH stabilizer is sodium citrate, citric acid, phosphate or acetate;
(4) diluting the resultant mixture in distilled water to a concentration of the pH stabilizer of 0.1 - 0.5 mg/ml and a concentration of Radix Panax Notoginseng saponins of 0.1mg - 14.0 mg RgI component/ml; filtering the solution untl clear, and therafter pasteurizing and packing the resulting product as Radix Panax Notoginseng saponins intravenous injection composition.

The present invention further provides compositions produced by such method which are of use for intravenous injection of saponins of *Radix notoginseng* in large doses with a stable pH, which will be used for prevention and treatment of cardiac and cerebral vascular disease and its sequelae.

For said saponins of *Radix notoginseng* intravenous injection, the preferred concentration is 1.4mg (calculated as Rg1)/ml. Said iso-osmotic preparation could be sodium chloride, glucose, sorbital and so on, among which the sodium chloride is preferred. The concentration of the sodium chloride falls between 7.5-9.5mg/ml among which 8.5mg/ml is preferred. Said pH stabilizer could be sodium citrate, citrate, phosphate, and acetate among which the sodium citrate is preferred. The concentration of the sodium citrate is 0.1-0.5mg/ml, in which 0.3mg/ml is preferred.

The invention therefore provides a means for large dose intravenous injection in which the active component is saponins of *Radix notoginseng*. This medicine is for prevention and treatment of cardiac and cerebrovascular disease and its sequelae. The concentration of the medicine is 0.1mg-14.0mg (calculated as Rg1) saponins of *Radix notoginseng* per ml.

The applied amount of the medicine could be adjusted according to the patient's age, weight and seriousness of the disease. The amount for each time is usually 140mg-350mg (saponins of *Radix notoginseng*). It could be intravenously injected 1-2 times per day for 28 days.

The animal experiments showed that the saponins of Radix notoginseng intravenous injection is a good protective medicinal treatment of cerebral anemia. It could significantly increase the cerebrovascular circulation and decrease its resistance, invigorate the circulation of blood, significantly improve rabbit eyeball conjunctiva micro-circulation aplastic status, and increase circulation and capillary openings. It has a good effect on treatment of embolismic cerebrovascular disease and could remove stases.

The method of production for the invention of saponins of *Radix notoginseng* intravenous injection is simple and convenient. It is also very practical and inspiring. In this method, the pH was stabilized by adding pH stabilizer to the saponins family of *Radix notoginseng* intravenous injection. This successfully solved the problem of decreasing pH, which could cause saponin hydrolysis and affect clarity of the intravenous injection preparation.

Further descriptions with experiments and diagrams for the invention follow.

### Brief Description of the Drawing

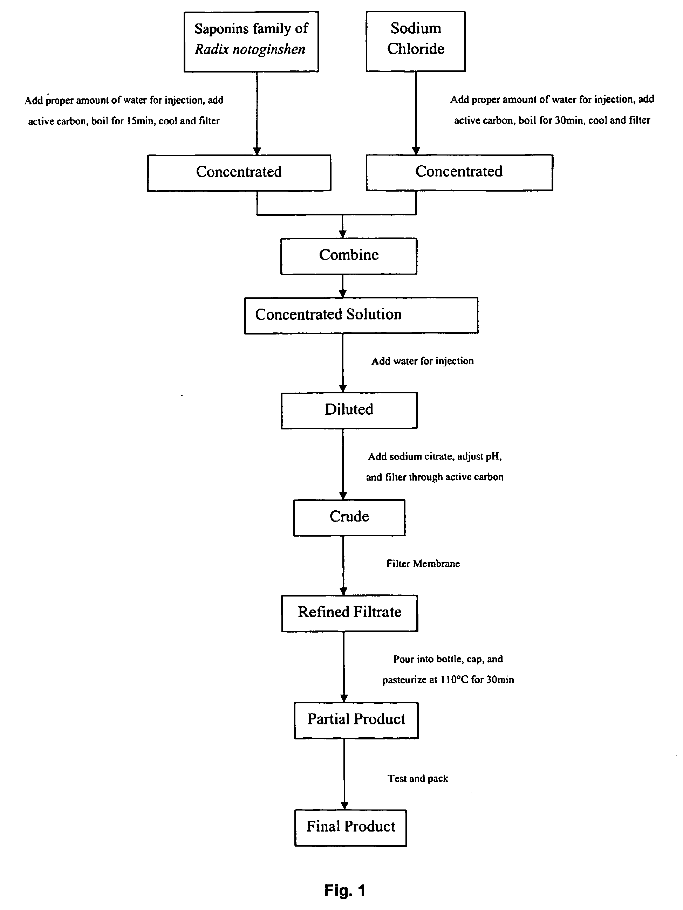

Figure 1 is the technological process diagram for the saponins family of *Radix notoginseng* intravenous injection.

### Detailed Description of Embodiments

Experiment 1. Preparation of the saponins family of *Radix notoginseng* intravenous injection.

### 1. Materials

| | |
|---|---|
| saponins family of *Radix notoginseng* (calculated as Rg1) | 0.1g |
| sodium citrate | 0.3g |
| sodium chloride | 7.5g |
| active carbon | 0.8g |
| distilled water | (added to) 1000ml |

### 2. Methods

The technological processes are shown as Figure 1. The production steps for the saponins family of *Radix notoginseng* intravenous injection are as follows. First, 7.5g of sodium chloride was dissolved in distilled water and diluted to a concentration of 100mg/ml. 0.4g of active carbon was added to the solution. The liquid was stirred, boiled, cooled, and filtered to be rid of active carbon. 0.1g (calculated as Rg1) of the saponins family of *Radix notoginseng* was then dissolved in the filtrate. Next, 0.3g of sodium citrate was added to the solution. The pH of the liquid was adjusted to 6.0 by adding sodium hydroxide and diluted to a volume of 1000ml. The solution was then filtered through 0.4 g active carbon, a 0.45µm filter membrane, and a 0.22µm filter membrane until clear. The final product of saponins family of *Radix notoginseng* intravenous injection was put in a 100 ml fluid infusion bottle, capped, pasteurized at 110°C, checked and then packed.

### Experiment 2. Preparation of the saponins family of Radix notoginshen intravenous injection.

### 1. Materials

| | |
|---|---|
| saponins family of *Radix notoginseng* (calculated as Rg1) | 1.0g |
| sodium citrate | 0.3g |
| sodium chloride | 8.5g |
| active carbon | 0.8g |
| distilled water | (added to) 1000ml |

### 2. Methods

The technological processes are shown in Figure 1. The production steps for the saponins family of *Radix notoginseng* intravenous injection are as follows. First, 8.5g of sodium chloride was dissolved in distilled water and diluted to a concentration of 100mg/ml. 0.4g of active carbon was added to the solution. The liquid was stirred, boiled, cooled, and filtered to be rid of active carbon. 1.0g (calculated as Rg1) of the saponins family of *Radix notoginseng* was then dissolved in the filtrate. Next, 0.3g of sodium citrate was added to the solution. The pH of the liquid was adjusted to 6.0 by adding sodium hydroxide and diluted to a volume of 1000ml. The solution was then filtered through 0.4g of active carbon, a 0.45µm filter membrane, and a 0.22µm filter membrane until clear. The final product of saponins family of *Radix notoginseng* intravenous injection was put in a 100ml fluid infusion bottle, capped, pasteurized at 110°C, checked and then packed.

### Experiment 3. Preparation of the saponins family of Radix notoginseng intravenous injection.

### 1. Materials

| | |
|---|---|
| saponins family of *Radix notoginseng* (calculated as Rg1) | 1.4g |
| sodium citrate | 0.3g |
| sodium chloride | 8.5g |
| active carbon | 0.8g |
| distilled water | (added to) 1000ml |

### 2. Methods

The technological processes are shown as Figure 1. The production steps for the saponins family of *Radix notoginseng* intravenous injection are as follows. First, 8.5g of sodium chloride was dissolved in distilled water and diluted to a concentration of 100mg/ml. 0.4g of active carbon was added to the solution. The liquid was stirred, boiled, cooled, and filtered to be rid of active carbon. 1.4g (calculated as Rg1) of the saponins family of *Radix notoginseng* was then dissolved in the filtrate. Next, 0.3g of sodium citrate was added to the solution. The pH of the liquid was adjusted to 6.0 by adding sodium hydroxide and diluted to a volume of 1000ml. The solution was then filtered through 0.4g of active carbon, a 0.45µm filter membrane, and a 0.22µm filter membrane until clear. The final product of saponins family of *Radix notoginseng* intravenous injection was put in a 100 ml fluid infusion bottle, capped, pasteurized at 110°C, checked and then packed.

### Experiment 4. Preparation of the saponins family of Radix notoginseng, intravenous injection.

### 1 Materials

| | |
|---|---|
| saponins family of *Radix notoginseng* (calculated as Rg1) | 3.5g |
| sodium citrate | 0.3g |
| sodium chloride | 8.5g |
| active carbon | 0.8g |
| distilled water | (added to) 1000ml |

### 2 Methods

The technological processes are shown as Figure 1. The production steps for the saponins family of *Radix notoginseng* intravenous injection are as follows. First, 8.5g of sodium chloride was dissolved in distilled water and diluted to a concentration of 200mg/ml. 0.4g of active carbon was added to the solution. The liquid was stirred, boiled, cooled, and filtered to remove active carbon. 3.5g (calculated as Rg1) of the saponins family of *Radix notoginseng* was then dissolved in the filtrate. Next, 0.3g of sodium citrate was added to the solution. The pH of the liquid was adjusted to 6.0 by adding sodium hydroxide, then diluted to volume of 1000ml. The solution was then filtered through 0.4g active carbon, a 0.45µm filter membrane, and a 0.22µm filter membrane until clear. The final product of saponins family of *Radix notoginseng* intravenous injection was put in a 100ml fluid infusion bottle, capped, pasteurized at 110°C, checked and then packed.

### Experiment 5. Preparation of the saponins family of Radix notoginseng intravenous injection

### 1. Materials

| | |
|---|---|
| saponins family of *Radix notoginseng* (calculated as Rg1) | 7.0g |
| sodium citrate | 0.3g |
| sodium chloride | 9.5g |
| active carbon | 1.0g |
| distilled water | (added to) 1000ml |

### 2. Methods

The technological processes are shown as Figure 1. The production steps for the saponins family of *Radix notoginseng* intravenous injection are as follows. First, 9.5g of sodium chloride was dissolved in distilled water and diluted to a concentration of 200mg/ml. 0.4g of active carbon was added to the solution. The liquid was stirred, boiled, cooled, and filtered to be rid of active carbon. 7.0g (calculated as Rg1) of the saponins family of *Radix notoginseng* was then dissolved in the filtrate. Next, 0.3g of sodium citrate was added to the solution. The pH of the liquid was adjusted to 6.0 by adding sodium hydroxide and diluted to volume of 1000ml. The solution was then filtered through 0.6g of active carbon, a 0.45µm filter membrane, and a 0.22µm filter membrane until clear. The final product of saponins family of *Radix notoginseng* intravenous injection was put in a 100ml fluid infusion bottle, capped, pasteurized at 110°C, checked and then packed.

### Experiment 6. The protective effect of the saponins family of Radix notoginseng intravenous injection to rat cerebral ischemia reperfusion injury.

(1) Experimental medicine: 5% saponins family of *Radix notoginseng* intravenous injection preparation (manufactured by Lizhu Group Limin Pharmaceutical Industry, packed as 100ml, batch No. 20000301). The total amount of saponins of *Radix notoginseng* intravenous injection preparation used in clinical practice is 350mg/250ml per treatment. It could be applied 1-2 times per day for 28 days for one treatment phase. The 5% saponins family of *Radix notoginseng* intravenous injection in the experiment was diluted to the needed concentration with physiological saline.
(2) Experimental animal: 150 SD rats with body weight of 280.45±14.91g, half male, half female.
(3) Amount of medicament: calculated for the experimental animal according to the one for human by using transform factor mg/Kg-mg/m². The rat low dosage group was given a single dose of the equivalent amount of medicament for animals. The medium dosage group was given twice the amount as the low dosage group, and the high dosage was given 4 times the amount of the low dosage group. The amount of saponins family of *Radix notoginseng* intravenous injection for an adult is 250ml per day (containing 350mg of saponins family of *Radix notoginseng*, which is 350mg/60kg per day. The equivalent amount medicament for rats is 350mg/60kg × 35/6 = 34.03mg of saponins family of *Radix notoginshen.* High dosage is 34.03 × 4 = 136.12mg/Kg, 34.03 × 2 = 68.06mg/Kg, 34.03 × 1 = 34.03mg/Kg.
   The control medicament was the Salvia Miltiorrhiza injection which is well used in present day Chinese clinical practices. The amount of medicament administered temporally for an adult is 10ml (containing 10g of crude drug), twice per day, which makes 20g of crude drug/60kg per day. The applying amount of Salvia Miltiorrhiza injection for a rat is 20g/60kg × 35/6 = 1.94g/Kg.
(4) Experimental conditions: room temperature (25°C), relative humidity 60% ~ 70%
(5) Experimental methods:
   150 SD rats were used, of which half were male and half were female, each of them weighting 282.11±14.77g. They were grouped into 6 groups with 6 rats per group. The first group was a pseudo-operation group. The second group was a model group. The third group was a Salvia Miltiorrhiza injection group. The fourth to the sixth groups were applied, respectively, with low dosage, medium dosage, and high dosage of saponins family of *Radix notoginshen* intravenous injection. Each group was applied with medicament through lingual veins continuously for 4 days. The first and second groups were injected with same amount of physiological saline. One hour after the last medicament application, the animals were anaesthetized using 1g/Kg ip of Urethane. Both sides of the a.carotis communis were then separated and threaded for future experiments. The animals were then injected with 50mg/Kg 5% Evans Blue (EB) and 486u/Kg heparin through v.femoralis. After 5 minutes, both sides of rat a.carotis communis in the model and other medicament groups were clipped shut. And at the same time, 2.5ml of blood was drained from the v.femoralis. These two steps above were not included in the pseudo-operation group. The a.carotis communis of rats in the model and other medicament groups were released and reinfused for another 30 minutes after 3 hours. The rats were then decapitated on ice and the brain (the bulbus olfactorius, cerebellum and low truncus encephalicus) carefully removed. Two rat brains were randomly selected from each group and fixed in formaldehyde and glutaraldehyde for morphological observation. The rest were weighed and soaked in 5ml of formamide, water bathed for 48 hours at 45°C, and regularly stirred. The supernatant was obtained and 1.5ml of chloroform was added. Next, the liquid was centrifuged and the supernatant was obtained and analyzed using an ultraviolet spectrophotometer (620nm). The content of Evans Blue in the rat brains was calculated using standard curves. The second rat brain taken was baked in the oven at 110°C until its weight was constant. The brains were then weighed and water content of the brains were calculated as % = (brain wet weight - brain dry weight) / brain wet weight × 100%, and brain index was calculated as (brain wet weight/100g body weight).
(6) Results
   1) The effect of saponins family of *Radix notoginseng* intravenous injection on experimental rat brain indexes, water content and Evans Blue content.
      The experimental results are shown in Figure 1. It shows that the brain indexes, water content and EB content of pseudo-operation group all decreased, and had significant statistical differences which indicated success in operation modeling. When comparing the model group with the three groups which were applied saponins family of *Radix notoginseng* intravenous injection, the above indexes all decreased and had significant statistical differences. These facts indicated that the saponins family of *Radix notoginseng* intravenous injection could improve incomplete ischemia reperfusion injury of the experimental animals, relieve the increase of cerebrovascular penetrating and cerebral edema caused by cerebral ischemia reperfusion injury, and thus protect the brain.
   2) The effect of saponins family of *Radix notoginseng* intravenous injection on experimental rat brain histological and morphological changes.

Results observed under the microscope are as follows. The results were normal for the pseudo-operation group. Occasional capillary hyperemia, slight expansion between vascular and nerve cells were observed. The results for the model group were quite different. Typical cell edema, denaturation and necrosis were observed. Also there was significant expansion between meningeal vascular, neurons, and cerebral cortex cells, expansion of cell nucleus, nucleolus clouding, disappearance of tiger porphyritics, bleeding of cerebral cortex, local congestion of colloidal cells, and occasionally cell ischemia necrosis. The symptoms included decreasing cell count, disappearance of the cell outlines and local hemorrhage. Different degrees of the cell edema and denaturation were also observed from the positive control group and three saponins family of *Radix notoginseng* intravenous injection groups. However, the symptoms for the positive control group and three medicament groups were far milder than that of the model group. The symptoms included expansion of the cavity between cells and vasculars and vascular hyperemia. Propagation of small colloidal cells was observed in the high dosage treatment group.

Results observed under the electron microscope are as follows. In the pseudo-operation group, clear cell stuctures, intact axon and cell membranes, evenly distributed chromosomes, clear mitochondria, clear Golgi body and clear rough endoplasmic reticulum were observed. All structures seemed to be normal.

In the model group, the abnormal symptoms observed included expansion of neuron cells, decreasing cell count, congestion of nuclear chromosomes, expansion of the Golgi body, appearance of empty bubbles in the Golgi body, expansion of rough endoplasmic reticulum, significant expansion of mitochondrion, obscure cristae, expansion of vascular peripheral cavities, destruction of the endomembrane, and partial dissolution of nuclear chromotin. In the three saponins family of *Radix notoginshen* intravenous injection groups and the positive control group, there was indication of ischemia edema, which is however, far less severe than that of the model group. The symptoms included congestion of the chromosomes, various degrees of expansion of the rough endoplasmic reticulum, Golgi body and mitochondria, but at levels much less severe than that of the model group.

According the results observed under the electron microscope, the most severe injuries were observed in model group. This indicated that the model is constructed successfully. On the other hand, much less severe damage was observed in the three saponins family of *Radix notoginseng* intravenous injection groups and the positive control group. Furthermore, the cell damage decreased while the amount of saponins family of *Radix notoginshen* intravenous injection increased. This indicated that the saponins family of *Radix notoginseng* intravenous injection could protect animal from acute incomplete ischemia reperfusion injury.

**Table 1: Effect of saponins family of Radix notoginseng intravenous injection on experimental rat ischemia reperfusion injury (n=20)**

| Group | Dosage (mg/Kg) | EB content (µg/g brain wet weight) | Brain index (g brain/100g body weight) | Brain water content(%) |
|---|---|---|---|---|
| Model | -------- | 12.10±0.56 | 0.64±0.02 | 81.17±0.60 |
| Pseudo-operation | -------- | 6.81±0.75*** | 0.55±0.02*** | 77.14±1.00*** |
| saponins family of *Radix notoginseng* | 1940 | 10.56±0.49*** | 0.64±0.01 | 80.09±0.73*** |
| Low dosage of saponins family of | | | | |
| *Radix notoginseng* Medium dosage of saponins | 34.03 | 9.81±0.39*** | 0.63±0.01* | 80.44±0.60** |
| Medium dosage of saponins | | | | |
| family of Radix notoginseng seng | 68.06 | 61±0.32*** | 0.60±0.01*** | 78.63±0.57*** |
| High dosage of saponins family of | | | | |
| *Radix notoginseng* | 132.12 | 9.55±0.31*** | 0.60±.01*** | 78.99±0.73*** |

| | | | | |
|---|---|---|---|---|
| * compared to model group p<0.05,** p<0.01, ***p<0.001 | | | | |

The above results prove that the saponins family of *Radix notoginseng* intravenous injection has certain protective effects on experimental rat acute incomplete ischemia reperfusion injury. It is worthy to have further application on clinical practices.

### Experiment 7, the effect of the saponins family of Radix notoginseng intravenous injection on the cerebrovascular of anaesthetized dog

(1) Experimental medicament and its dosage: 5% saponins family of *Radix notoginseng* intravenous injection preparation (manufactured by Lizhu Group Limin Pharmaceutical Industry, packed as 100ml, batch No. 20000301). ). The total amount of saponins of Radix notoginseng intravenous injection preparation used in clinical practice is 350mg/250ml per treatment. It could be applied 1-2 times per day for a 28 day treatment phase. In the experiment, the dosage of the medicament for the experimental animal was calculated according to that for a human by using the transform factor mg/Kg-mg/m². A single dose of the equivalent amount of medicament for animals was applied to the dog low dosage group. The medium dosage group received twice the equivalent medicament and high dosage received 4 times the equivalent medicament of the low dosage group. The dosage of saponins family of *Radix notoginseng* intravenous injection for an adult is 250mln once per day. That is, 350mg of saponins family of *Radix notoginseng* is applied per day. In the case of dog, 1) the equivalent amount of medicament is 350mg/60kgx35/19=10.75mg saponins family of *Radix notoginseng* 2) low dosage is 10.75x1=10.75mg/Kg, 3) medium dosage is 10.75x2=21.5mg/Kg, 4) high dosage is 10.75x4=43mg/Kg. 5% of total amount of saponins of *Radix notoginshen* intravenous injection and Mailuoning injection were diluted before usage to a needed concentration using physiological saline.
The control medicament was Mailuoning injection (manufactured by Nanjing Jinglin Pharmaceutical Corp., medicine content of 10g/ml, batch No. 960812). The adult dosage was 10ml (crude drug of 10g), twice per day, that is, 20g/60kg per day. For the dog, 1) equivalent dosage is 20g/60kgx35/19=0.61 g/Kg, 2) dosage of medicament is 0.61x4=2.44 g/Kg. Physiological saline was manufactured by Southern Hospital Pharmacy, batch No. 20000926.
(2) Experimental animals: 30 healthy adult hybrid dogs, each weights 11-15kg, half male and half female.
(3) Experimental conditions: room temperature 25°C, relative humidity 60%~70%, and regular pasteurization of experimental equipments.
(4) Equipment: electromagnetic flowmeter, model FM-27, manufactured by Japanese Photoelectric Ltd. The [8] Physiological Recording Instrument of model RM-6000, manufactured by Japanese Photoelectric Ltd and Chengdu Instrument Industry.
(5) Method of medicaments application: intravenous injection with a concentration of 30ml/Kg.
(6) Experimental control: the negative control group was given same amount of physiological saline while the positive control group was given 4 times of the equivalent amount of the Mailuoning injection.
(7) Experimental data statistics
The experimental data was analyzed by statistical t-testing. The physiological statistics were used for data analysis for comparison among all groups and the negative control group before and after medicament applications.
(8) Experimental methods
The experimental animals were randomly grouped into 5 groups including the negative and positive control groups, and the low dosage, medium dosage and high dosage of saponins family of *Radix notoginshen* intravenous injection. The dogs were first anaesthetized via an intravenous injection of 30mg/Kg 3% pentobarbital sodium, and then fixed on the operation platform. 1mg/ml of pentobarbital sodium diluted in physiological saline was intravenously infused to maintain the anesthesia. The dog neck was cut for about 10cm to separate the left a.carotis communis, and thread on the same side the a.carotis externa and the a.vertebralis. The No. 4 sensor of the electromagnetic flowmeter was put in the a.carotis communis. The blood flow of the a.carotis communis, calculated by the electromagnetic flowmeter, could be considered as cerebral blood flow. At the same time, the a.fermoralis and v.fermoralis on one side were separated. The a.fermoralis was connected to the pressure transducer and v.fermoralis was connected to the 3-way pipe for medicament application. All four legs were connected to the sensors for the electrocardiogram monitor. Meanwhile, blood pressure of the a fermoralis, II lead electrocardiogram, and the average blood flow of the a.carotis communis were recorded by the [8] Physiological Recording Instrument. When the average blood flow, electrocardiogram and blood pressure were indicated stable by the equipment, the data were recorded and the medicaments were applied through intravenous injection. The average amount of medicament application was 30ml/Kg for all groups. At the same time, the cerebral blood flow, blood pressure and cardiac rhythm were recorded at 0min, 5min, 15min, 30min and 60min after medicament application. At the end of the experiment, the skull was opened and the whole brain was removed from above the medulla oblongata. The brain was then weighed. The brain weight of one side was obtained by dividing the resulting data by 2. The cerebral blood flow per minute for each gram of brain tissue was obtained by dividing the recorded cerebral blood flow by the brain weight of one side.
(9) Result
1) The effect of the saponins family of *Radix notoginshen* intravenous injection on the cardiac rhythm of anaesthetized dog
The experimental results indicated statistically meaningful fluctuations of the cardiac rhythm in the low dosage group recorded after medicament application 5min and 15min, and the medium dosage group recorded after 0min. There were no statistical differences in other groups and time phases (table 2).
2) The effect of the saponins family of *Radix notoginshen* intravenous injection on the blood pressure of anaesthetized dog
As in tables 3, 4, 5, systolic pressure decreases (p<0.05) in the anaesthetized dog was observed in the saponins family of *Radix notoginseng* intravenous injection groups 15min after application, while the effect on diastolic pressure was not obvious (the only statistical meaningful blood pressure decrease lay in the low dosage group at 5min after application). Also, the only statistical meaningful decrease of the average blood pressure was observed in medium and low dosage groups while no obvious change was observed in the high dosage group after medicament application.

**Table 2. The effect of the saponins family of Radix notoginseng intravenous injection on the cardiac rhythm of anaesthetized dog (beats/min, X±SD)**

| | medicament | | | medicament | application | |
|---|---|---|---|---|---|---|
| Before | application | | After | | | |
| | | 0min | 5min | 15min | 30min | 60min |
| Negative control | 212±13.3 | 213.5±10.7 | 213±12.7 | 211.8±12.4 | 209±11 | 209.5±12 |
| Positive control | 202.2±18.35 | 195.2±17.51 | 196.2±15.56 | 195±16.67 | | 191.4±15.06 191.2±12.87 |
| Low dosage | 204±20.86 | 204.67±21.22 | 178.67± | 178.33± | 189.33± | 202.67± |
| group | | | 17.87'" | 15.74'" | 14.68 | 17.99 |
| Medium dosage | 193.83±17.85 | 189.5±20.80' | 193.33±27.75 | 194.33±24.68 | 195.5±21.87 | 193.83±21.8 |
| group | | | | | | 9 |
| High dosage | 200.83±13.83 | 178.5±28 | 181.33±32.78 | 198.17±12.17 | 195.83±9.5 | 198.67±15.1 |
| group | | | | | | 1 |

Compared before and after medicament application, *P<0.05, **P<0.01
Compared with the negative control group, #P<0.05, **P<0.01

**Table 3. The effect of the saponins family of Radix notoginseng intravenous injection on the systolic pressure of anaesthetized dog (Kpa, X±SD)**

| Before | medicament | | | medicament | application | |
|---|---|---|---|---|---|---|
| | application | | After | | | |
| | | 0min | 5min | 15min | 30min | 60min |
| Negative control | 20.8±0.9 | 21.1±0.9 | 21.0±0.9 | 20.8±0.9 | 20.6±0.6 | 20.7±0.8 |
| Positive control | 21.1±0.8 | 18.5±2.5 | 20.1±0.8 | 20.7±0.7 | 20±0.8 | 20.5±0.5 |
| Low dosage group | 20.7±1.3 | 18.1±2.3 | 14.1±4.2'" | 15.4±4.5'' | 18.7±3.3 | 20.9±1.4 |
| Medium | dosage21.3±1.6 | 16.4±2.7'" | 17.7±2.9 | 20.3±1.1 | 21.1±1.0 | 21.2±1.4 |
| group | | | | | | |
| High dosage group | 21.2±1.4 | 15.3±3.5** | 19.5±2.3 | 20.9±1.3 | 20.9±1.3 | 21.5±1.0 |

Compared before and after medicament application, *P<0.05, **P<0.01

Compared with the negative control group, #P<0.05, ^{##}P<0.01

**Table 4. The effect of the saponins family of Radix notoginseng intravenous injection on the diastolic pressure of anaesthetized dog (Kpa, X±SD)**

| | Medicament | | After | Medicament | Application | |
|---|---|---|---|---|---|---|
| Before | Application | 0min | 5min | 15min | 30min | 60min |
| Negative control | 13.5±1.2 | 13.6±1.1 | 13.6±1.1 | 13.3±1.2 | 13.3±1.1 | 13.4±1.1 |
| Positive control | 13.2±1.4 | 11.5±2.0 | 12.8±1.8 | 13.5±1.6 | 13±0.8 | 13.3±1.2 |
| Low dosage group | 14.0±1.6 | 12.6±22 | 8.7±3.4** | 10.1±3.4 | 11.3±2.8 | 13.6±1.0 |
| Medium dosage group | 14.3±1.5 | 11.0±2.6 | 12.0±2.4 | 13.4±1.0 | 13.4±1.1 | 13.9±1.5 |
| High dosage group | 14.4±1.9 | 9.9±4.6 | 12.8±2.2 | 14.2±1.9 | 14.0±1.5 | 14.2±1.6 |

Compared before and after medicament application, *P<0.05, **P<0.01
Compared with the negative control group, #P<0.05, ^{##}P<0.01

**Table 5. The effect of the saponins family of Radix notoginseng intravenous injection on the average blood pressure of anaesthetized dog (Kpa, X±SD)**

| Before | Medicament | | After | Medicament | Application | |
|---|---|---|---|---|---|---|
| | Application | 0min | 5min | 15min | 30min | 80min |
| Negative control group | 16.3±1.0 | 16.6±1.1 | 16.6±1.0 | 16.6±0.9 | 16.3±1.2 | 16.4±1.1 |
| Positive control group | 16.2±1.1 | 14.1±1.9 | 15.7±1.2 | 16.0±1.2 | 15.7±0.5 | 15.9±0.8 |
| Low dosage group | 16.1±1.5 | 15.3±1.8 | 10.7±3.5*^{##} | 12.4±3.8 | 14.1±2.4 | 16.9±1.0 |
| Medium dosage group | 17.5±1 | 13.6±2.7* | 14.2±2.3* | 16.1±1.3 | 16.3±1.0 | 16.6±1.2 |
| High dosage group | 16.6±1.1 | 12.3±4.1 | 15.5±2.3 | 16.8±1.5 | 17.0±1.4 | 17.3±1.1 |

Compared before and after medicament application, *P<0.05, **P<0.01
Compared with the negative control group, #P<0.05, ^{##}P<0.01
3) The effect of the saponins family of *Radix notoginseng* intravenous injection on the cerebral blood flow of anaesthetized dog
Data of table 6 indicated that blood flow immediately increased (P<0.05) immediately after medicament application in all dosage groups of the saponins family of *Radix notoginseng* intravenous injection. The blood flow increase was maintained for 5min in the low and medium dosage group while it was maintained for 15min in the high dosage group. The blood flow dropped to the level before medicament application afterwards.

**Table 6. The effect of the saponins family of Radix notoginseng intravenous injection on the cerebral blood flow of anaesthetized dog (ml/g/min, X±SD)**

| | Medicament | | | | Application | |
|---|---|---|---|---|---|---|
| Before | Application | | After | Medicament | | |
| | | 0min | 5min | 15min | 30min | 60min |
| Negative control | | | | | | |
| group | 1.83±0.17 | 1.84±0.17 | 1.85±0.17 | 1.87±0.20 | 1.93±0.22 | 1.85±0.17 |
| Positive control group 1.78±0.14 | | 2.23±0.15*** | 2.21±0.14*** | 2.12±0.09*** | 2.07±0.09* | 2.00±0.12 |
| Low dosage group | 1.92±0.22 | 2.26±0.22** | 2.23±0.21* | 1.94±0.21 | 1.96±0.17 | 2.08±0.26 |
| Medium dosage group 1.62±0.21 | | 2.34±0.13**** | 2.28±0.09**** | 2.10±0.17 | 1.91±0.22 | 1.91±0.20 |
| High dosage group | 1.85±0.25 | 2.33±0.27** | 2.27±0.25** | 2.24±0.23** | 2.00±0.33 | 1.97±0.36 |

Compared before and after medicament application, *P<0.05, **P<0.01
Compared with the negative control group, #P<0.05, ^{##}P<0.01
4) The effect of the saponins family of *Radix notoginseng* intravenous injection on the cerebral vascular resistance of anaesthetized dog
Data from table 7 indicates that the cerebrovascular resistance decreased
(P<0.01) immediately after medicament application in all dosage groups. This effect was maintained for 15min after medicament application and then dropped to the level before application afterwards (P>0.05).

**Table 7. The effect of the saponins family of Radix notoginseng intravenous injection on the cerebral vascular resistance of anaesthetized dog (ml/g/min, X±SD)**

| Before | Medicament | | After | Medicament | Application | |
|---|---|---|---|---|---|---|
| | Application | 0min | 5min | 15min | 30min | 60min |
| Negative control group | 9.06±0.56 | 9.07±0.61 | 9.04±0.64 | 8.97±0.76 | 8.58±0.86 | 8.89±0.60 |
| Positive control group | 9.10±0.54 | 8.33±0.81**** | 7.13±0.32**** | 7.37±0.47*** | 7.61±0.27** | 7.99±0.27* |
| Low dosage group | 8.43±0.35 | 8.79±0.82**** | 4.91±1.68**** | 6.43±2.02* | 7.35±1.60 | 8.33±1.30 |
| Medium dosage group | 9.82±1.38 | 5.78±0.91**** | 6.22±0.90 | 7.71±0.79* | 8.67±1.11 | 8.81±0.92 |
| High dosage group | 9.13±0.91 | 5.20±1.42*** | 6.84±0.93**** | 7.58±0.62** | 8.79±1.48 | 9.22±1.78 |

Compared before and after medicament application, *P<0.05, **P<0.01
Compared with the negative control group, #P<0.05, ^{##}P<0.01
(10) Conclusion

The saponins family of *Radix notoginseng* intravenous injection caused decreases in the cardiac rhythm and blood pressure for the anaesthetized dog within a short period of time after application. However, this effect did not become more significant when amount of medicament increased. The saponins family of *Radix notoginshen* intravenous injection also significantly increased the cerebral blood flow and decreased the cerebral vascular resistance for the experimental animals.

The two effects mentioned above also recovered to the level before medicament application afterwards. Experiment 8, the effect of the saponins family of *Radix notoginseng* intravenous injection on the hemato-rheology and micro-circulation of anaesthetized dog

### (1) Experimental medicament

5% of the saponins family of *Radix notoginseng* intravenous injection preparation, manufactured by Lizhu Group Limin Pharmaceutical Industry, packed as 100ml, batch No. 20000301. The application amount in clinical practices is an intravenous infusion of 350mg/250ml (calculated as the saponins family of *Radix notoginshen*)*.* It was applied 1-2 times per day for 28 days to complete one phase. The experimental medicaments were diluted with physiological saline to a concentration of 2.5%, 1.25% and 0.63%. Medicaments were stored in the refrigerator at 4°C The positive medicament (the saponins family of *Radix notoginseng* intravenous injection) contained 2ml/shot. Each shot contained 1g of root of red rooted salvia and *Lignum Dalbergiae Odoriferae* respectively, manufactured by Guangdong Yongkang Pharmaceutical Ltd., batch No. 00070001. The experimental physiological saline was prepared to the desired concentration and stored at 4°C in the refrigerator.

The main preparations were polymerized dextranum with molecular weight of 500,000, made in Sweden, Sodium adenosine diphosphate (ADP) manufactured by FARCO, Sodium heparin provided by Chinese Pharmaceutical Corp. Beijing Branch, Urethane provided by Shanghai Chemical Preparation Store, and pentobarbitol sodium provided by Guangzhou Chemical Industry.

### (2) Experimental animals

Kunming breed mice, each weighing 18-24g, in a 1:1 male to female ratio; SD rats, each weighing 190-260g, in a 1:1 male to female ratio; New Zealand rabbit, each weighing 2.0-2.5kg, both male and female. The males and females were separated. Each cage contained one rabbit/5 mice/5 rats. The animals were kept 3 days for adaptation. They were only used in experiments after no abnormal physical behaviors or physiological symptoms were observed. The animals were well fed and the room was well lighted and aired. Temperature was maintained at 20-25°C. Humidity was 45-65%. Professional management and regular pasteurization were applied to the room.

### (3) Dosage set up for the saponins family of Radix notoginseng intravenous injection

The drug form of the saponins family of Radix notoginseng intravenous injection in clinical practices is sodium chloride intravenous instillation. The dosage for adult is 350mg (the saponins family of Radix notoginseng per treatment, 1-2 treatments per day. According to the LD₅₀ and toxicity experiment results of this drug, the adult dosage applied in this experiment was 350mg per day. A transform factor was used to calculate the dosage for experimental animals according to that for humans. The equivalent dosage for animals was calculated using this transform factor. The low dosage group received a single dose of the equivalent dosage. The medium dosage group received twice the equivalent dosage. And the high dosage group received 4 times the equivalent dosage. The salvia miltiorrhiza injection was used as the positive control with the equivalent dosage.
1) Dosage for mice: equivalent dosage for mice = 350/60×35/3=68mg/Kg; low dosage for mice =68×1=68mg/Kg; medium dosage for mice =68×2=136mg/Kg; high dosage for mice =68x4=272mg/Kg.
2) Dosage for rats: equivalent dosage for rats = 350/60x35/6=34mg/Kg; low dosage for rats =34×1=34mg/Kg; medium dosage for rats =34×2=68mg/Kg; high dosage for rats =34×4=136mg/Kg.
3) Dosage for rabbits: equivalent dosage for rabbits 350/60×35/12=17mg/Kg; low dosage for rabbits =17×1=17mg/Kg; medium dosage for rabbits =17×2=34mg/Kg; high dosage for rabbits =17x4=68mg/Kg.
4) Dosage of salvia miltiorrhiza injection for positive control group: adult dosage in clinical practice = 20ml per day using intravenous instillation; equivalent dosage for mice = 20/60×35/3=3.9ml/Kg=4ml/Kg; equivalent dosage for rats = 20/60×35/6 = 1.94ml/Kg = 2ml/Kg; equivalent dosage for rabbits
   =20/60×35/12=0.97ml/Kg=1 ml/Kg.
5) Blank control group: equivalent amount of physiological saline was applied.

### (4) Medicament application methods

The medicine was applied through intravenous injection (IV) the same as in clinical practice. The amount of application was 13ml/Kg for mice, 3.2ml/Kg for rats and 1.6ml/Kg for rabbits.

### (5) Experimental Procedures

### 1) Mice blood coagulating time testing (slide test)

50 Kunming breed mice were chosen in a 1:1 male to female ratio. Each weighed 18-24g. Animals were randomly grouped into 5 groups: bland control group, positive control group and 3 medicament groups respectively applied with low dosage, medium dosage and high dosage of the saponins family of *Radix notoginshen* intravenous injection. Mice of each group received medicament through tail intravenous injection of the indicated amount continuously for 3 days. 30min after the last application of medicament, two drops of blood were obtained from the plexus venosus behind the eyeball. Each blood drop was set on a slide and has a diameter of 5mm. Time was monitored immediately after blood dropped on the slide. Every 30s, a clean No. 4 needle was used to stir the blood drop once from the edge to the center, to observe whether there appeared any blood filiform. The time period from the obtaining of blood to the appearance of blood filiform was considered as blood coagulation time. The second drop of blood was used for retesting. The experimental results are shown in Table 8. The data indicates that the blood coagulation time for the medicament application groups was significantly longer than that for the blank control group. The statistic analysis results indicated remarkable differences: P<0.05 or P<0.01. The anti-coagulation effect of the saponins family of *Radix* notoginserg intravenous injection increased with increasing dosage. However, there was no significant difference between the 3 different dosage groups.

**Table 8. Effect of medicaments on blood coagulation time (n=10)**

| Group | Medicament and Dosage | Coagulation Time |
|---|---|---|
| Blank Control | Physiological Saline , 13ml/Kg | 95.9±19.59 |
| Positive Control | Salvla Mittiorthiza Injection , 4 ml/Kg | 128.8±30.64* |
| | 0.63% Saponins family of *Radix notoginshen* | |
| Low Dosage Group | intravenous injection , 68mg/Kg | 120.4±31.35 |
| | 1.25% Saponins family of *Radix notoginshen* | |
| Medium Dosage Group | intravenous injection 136mg/Kg | 128.0±25.33* |
| High Dosage Group | 2.5% Saponins family of *Radix notoginshen* | 137.2±29.29** |
| | intravenous injection, 272mg/Kg | |

| | | |
|---|---|---|
| *P <0.05, **P<0.01, compared to the blank control group | | |

### 2) Rat thrombosis experiment in vivo

50 Kunming breed rats were chosen in a 1:1 male to female ratio. Each weighed 220-280g. Animals were randomly grouped into 5 groups: bland control, positive control, and 3 medicament groups applied with low dosage, medium dosage and high dosage, respectively, of the saponins family of *Radix notoginseng* intravenous injection. Rats of each group received medicament through tail intravenous injection of the indicated amount continuously for 2 days. Thrombosis *in vivo* experiments were conducted on the 3^{rd} day after medicament application. Animals were anaesthetized by 20% Urethane (1g/Kg, IP). The neck was cut and the right side a.carotis commubis and the left side of v.jugularis externa were separated. A No. 4 operation thread of 5cm was set in the middle pipe of the three connected polyethylene pipes. Among the 3 pipes, two at both ends had an internal diameter of 1 mm and a length of 10cm while the middle one had an internal diameter of 2mm and a length of 8cm. The pipes were filled with heparin physiological saline (50u/ml). The heparin (50u/Kg) was filled from the left side of v.jugularis externa through the pipe. Then the pipe was clipped and inserted from the other end to right side of a.carotis commubis. Said amount of medicament was injected through the v.jugularis externa after the operation. The circulation was released 5min after medicament application. The blood was then shunted from the right side of a.carotis commubis to the left side of v.jugularis externa. Circulation was again halted after 15min. The pre-set operation thread was removed and weighed. The thrombus wet weight was obtained by subtracting thread weight from the total weight. The resistant ratio was calculated according to the following equation:
Resistant ratio (%) = (thrombus weight from the control group - thrombus weight from medicament application group) ÷ thrombus weight from control group × 100%

The experimental results were shown as in Table 9. The data indicates that the thrombus wet weight from the medicament application group was obviously lower than that from the blank control group. The statistical analysis showed significant differences of P<0.05 or P<0.01. The anti-coagulation effect of the saponins family of *Radix* notoginseng intravenous injection increased with increasing dosage. However, there was no obvious difference between 3 different dosage groups.

**Table 9. Medicament effect on rat thrombosis (n=10)**

| Group | Medicament and Dosage | Thrombosis wet weight | Resistant ratio |
|---|---|---|---|
| | | (mg) | (%) |
| Blank control | Physiological saline, 3.2ml/Kg | 27.2±5.65 | |
| Positive control | Salvia Miltiorrhiza Injection, 2ml/Kg | 16.6±5.33** | 39.0 |
| Low dosage group | 1.25% Saponins family of Radix notoginseng | 21.9±495* | 19.5 |
| | intravenous injection, 34mg/kg | | |
| Medium dosage | 2.5% Saponins family of notoginseng | 21.1±6.61* | 22.4 |
| group | intravenous injection, 68mglKg | | |
| High dosage group | 5% Saponins family of *Radix* notoginseng 19.3±5.38* 29.0 | | |
| | intravenous injection, 136mg/Kg | | |

| | | | |
|---|---|---|---|
| *P <0.05, **P<0.01, compared to the blank control group | | | |

### 3) Testing of rat blood viscosity, hematocrit and platelet aggregation ratio

50 SD rats were chosen in a 1:1 male to female ratio. Each weighed 220-280g. Animals were randomly grouped into 5 groups: bland control, positive control, and 3 medicament groups applied with low dosage, medium dosage, and high dosage, respectively, of the saponins family of *Radix* notoginseng intravenous injection. Rats of each group were applied medicament through tail intravenous injection of the indicated amount continuously for 3 days. The animals were anaesthetized after 15min. by 20% Urethane (1g/Kg, IP). Blood was obtained from rat aorticus abdominalis. 4ml of blood was first obtained together with 1% heparin for anti-coagulant (150µl heparin:4ml blood). The two above mentioned preparations were used in testing of whole blood viscosity, plasma viscosity, and hematocrit. Another 4ml of blood was obtained together with 3.8% sodium citrate anti-coagulant (1:9). The above mentioned two preparations were used in platelet aggregation ratio test.

The instrument used in testing of blood viscosity was LG-R-80A automatic washing blood viscosity instrument manufactured by Beijing Shidi Scientific Instrument Corp. The whole blood viscosity and plasma viscosity were tested respectively at 150, 30, 5, 1 S⁻¹. The operation was carried out according to the instrument description.

For hematocrit testing, 1ml of whole blood was filled in RBC cuvette and centrifuged at 3000rmp for 30min. The height of the red blood cell column was recorded. The formula for determining percent hematocrit follows:
hematocrit = height of red blood cell column ÷ height of whole blood column × 100%

For platelet aggregation ratio testing, the blood with 3.8% sodium citrate added was centrifuged at 800rpm for 6min. The supernatant was platelet replenish plasma (PRP). The rest of the blood was re-centrifuged at 3000rpm for 10min after the supernatant was removed. This time the supernatant was platelet poor plasma (PPP). The blood was induced using ADP (5×10⁻⁶mol/L). The rat platelet aggregation ratio within 5min was tested using PABER-1 platelet aggregation instrument manufactured by Beijing Shidi Scientific Instrument Corp. The operation was carried out according to the instrument descriptions.

The experimental results are shown in Tables 10 and 11. Table 10 indicates that rat whole blood viscosity, hematocrit and plasma viscosity were significantly decreased in the saponins family of *Radix* notoginseng intravenous injection high dosage group. However, rat whole blood viscosity, hematocrit, and plasma viscosity showed no obvious changes in the saponins family of *Radix* notoginseng intravenous injection low and medium dosage groups. The salvia miltiorrhiza injection only had effect on whole blood viscosity at low shear rate (5 s⁻¹ and 1 s⁻¹) while it had no effect on other indexes. Table 11 indicates that the rat platelet aggregation ratio obviously decreased in the three different experimental medicament groups and the positive control group compared to that in the blank control group. The differences showed high significance (P<0.01) according to statistic analysis. The anti-coagulation effect of the saponins family of *Radix* notoginseng intravenous injection increased with the increase in dosage. There were significant differences between the high dosage and low and medium dosage group (P<0.01). However, there were few differences between the low and medium dosage group.

**Table 10. Effect of medicament on rat blood viscosity and hematocrit (n=10)**

| | Whole blood viscosity | | | | | | hematocrit(%) |
|---|---|---|---|---|---|---|---|
| Group | plasma viscosity (%) | | | | | | |
| | 150 s⁻¹ | 30 s⁻¹ | 5 s⁻¹ | 1 s⁻¹ | | | |
| Blank control | 6.9±0.79 | 8.5±0.64 | 11.5±1.29 | 18.9±2.61 | 2.2±0.83 | 45.3±4.88 | |
| Positive control | 6.8±0.93 | 7.8±1.00 | 10.0±1.40* | 15.9±2.85* | 1.8±0.36 | 45.9±4.01 | |
| Low dosage group | 7.1±1.07 | 8.3±1.05 | 10.7±1.71 | 17.5±2.80 | 1.6±0.43 | 44.3±3.20 | |
| Medium dosage group | 7.4±0.85 | 8.5±0.91 | 11.2±1.33 | 17.8±2.38 | 1.8±0.21 | 47.7±2.06 | |
| High dosage group | 5.8±0.69* | 7.0±0.73* | 9.0±1.04* | 13.8±1.92* | 1.6±026* | 40.0±4.47* | |

| | | | | | | | |
|---|---|---|---|---|---|---|---|
| *P <0.05, compared to the blank control group | | | | | | | |

**Table 11. Effect of medicament on rat platelet aggregation ratio (n=10)**

| Group | Medicament And Dosage | Maximum aggregation (% ) |
|---|---|---|
| Blank control | Physiological Saline, 3.2ml/Kg | 86.2±4.43 |
| Positive control | | 48.9±7.14** |
| Low dosage group | Salvia miltiorrhiza injection, 2ml/Kg | 68.1±1.10**^{##} |
| | 1.25% Saponins family of *Radix notoginshen* | 62.6±8.58**^{##} |
| Medium dosage | intravenous injection, 34mg/Kg | 47.9±7.88** |
| group | 2.5% Saponins family of *Radix notoginshen* | |
| High dosage group | intravenous injection, 68mg/Kg | |
| | 5% Saponins family of *Radix notoginshen* intravenous | |
| | injection, 136mg/Kg | |

| | | |
|---|---|---|
| **P <0.01, compared to the blank control group. ##P<0.01, compared to the high dosage group | | |

### 4) Observation of rabbit eyeball conjunctiva microcirculation

30 New Zealand rabbits were chosen in a 1:1 male to female ratio. Each weighed 2.0-2.5kg. Animals were randomly grouped into 5 groups (6 in each group): blank control, positive control, and 3 medicament groups applied with low dosage, medium dosage and high dosage, respectively, of the saponins family of *Radix notoginseng* intravenous injection. Rabbits were anaesthetized using 3% Sodium Pentobarbitol (30mg/Kg, IV). The animals' left eye lids were then opened and the eyeball conjunctiva microcirculation was observed using MTV-3801CB microcirculation micro video recorder system (provided by Shanghai Laser Technology Research Institute). The magnifying factor used was 2800X after the recorder was standardized using 0.01 mm objective lens micrometer (made by Shanghai 3^{rd} Industry of Optical Instruments). The general flow of the normal eyeball conjunctiva microcirculation was observed. Then 10% polymer dextranum (6ml/Kg) was intravenously injected through rabbit ear vein. The injection induced the acute microcirculation obstacle and changes in the microcirculation were observed. After 15min, one of three solutions was applied through intravenous injection: physiological saline, the Saponins family of *Radix notoginshen* injection, or the salvia miltiorrhiza injection. The applied medicament volume was 1.3ml/Kg for each medicament or saline. The same area of microcirculation was observed 15min after medicament application.

### Observation Indexes

Red blood cell fluid state: According to relative articles and experimental results from this invention, the red blood cell fluid state was classified into 6 levels: linear flow, linear granular flow, granular linear flow, granular flow, stasis, and stop.

Capillary net conjunction: The edge of the observation area was composed of small arteries and veins. The number of conjunctions between capillaries in this area and the capillaries on the edge was calculated. The non-conjunctive capillaries were not counted.

The results are shown in Table 12. The data indicated that the blood flow in rabbit eyeball microcirculation changed from normal linear or linear granular flow into granular or granular linear flow after being injected with 10% polymer dextranum. Also, the flow speed significantly decreased. Stasis and stop occurred in a few cases. However, no obvious thrombus or blood vessel distortion was observed. There was no significant blood flow changes observed when injected with physiological saline. After being injected with the saponins family of *Radix notoginshen* or the salvia miltiorrhiza, the microcirculation flow speed was greatly increased, flow state was mostly linear granular, and capillaries conjunctions were also increased. The increases were significant (P<0.05 or P<0.01) in the dosage group of saponins family of *Radix notoginseng* and positive control group comparing the states before and after medicament application.

**Table 12. Medicament effect on rabbit eyeball microcirculation (n=6)**

| Group | Blood Flow State | | Capillary Conjunctions | |
|---|---|---|---|---|
| | Before medicament | After medicament | Before | After medicament |
| Blank Control | Granular Flow | Granular Flow | 3.0±0.89 | 2.7±0.82 |
| Positive Control | Granular Flow | Granular Linear Flow | 2.5±0.84 | 3.5±0.55^{*#} |
| Low Dosage Group | Granular Flow | Granular Linear Flow | 3.0±0.89 | 2.8±0.75 |
| Medium Dosage Group | Granular Flow | Granular Linear Flow | 2.7±0.81 | 4.0±1.41 |
| High Dosage Group | Granular Flow | Granular Linear Flow | 2.3±1.03 | 4.2±1.17^{*#} |

| | | | | |
|---|---|---|---|---|
| *P<0.01, compared to the blank control group ^{#} P<0.05, ^{##} P<0.01 compared to pre-medicament application | | | | |

### (6) Statistical Analysis

All experimental data was presented as x±s, and analyzed using t test and one way analysis of variance.

### (7) Results

The results of this experiment indicated that the saponins family of *Radix notoginseng* intravenous injection could significantly invigorate the circulation and remove stases. It could resist rat thrombosis and elongate the coagulation. The high dosage group had relatively greater remarkable effects on rat blood viscosity and hematocrit. It also could greatly resist rat platelet aggregation, resist the rabbit eyeball microcirculation obstacles, increase the capillary openings, and speed up the blood flow. The effect of the saponins family of *Radix notoginshen* intravenous injection showed certain dosage dependence within the experiment application dosage. However, no obvious differences were observed within high, medium, and low dosage groups.

Experiment 9, the pH stability test for the intravenous injection of this invention The experiment results of pH are shown in Table 13.
Sample A: Xueshuangtong injection, manufactured by Neimenggu Ganqika Pharmaceutical Industry.
Sample B: Intravenous injection of this invention, manufactured by Lizhu Group Liming Pharmaceutical Industry

| Batch No. (Sample A) | PH | Batch No. (Sample B) | PH |
|---|---|---|---|
| 99110411 | 4.46 | 20000305 | 6.06 |
| 2000010011 | 4.04 | 20000414 | 6.00 |
| 2000011413 | 4.64 | 20000542 | 5.72 |
| 2000012712 | 4.36 | 20000544 | 6.16 |
| 2000040512 | 5.48 | 20000647 | 6.16 |
| 2000061212 | 5.02 | 20000719 | 6.34 |
| 2000072811 | 5.37 | 20000859 | 6.48 |
| 2000072812 | 5.69 | | |
| 2000090811 | 5.10 | | |

The Chinese Medicine standard specified that the qualified pH range of Xueshuantong injection was 5.0-7.0. The experimental results indicated that the pH of sample A was related to the batch No. This showed the instability of sample A pH, which decreased after storage. On the other hand, the pH of Sample B was more stable.

## Claims

1. A method for preparing a composition for intravenous injection comprising Radix Panax Notoginseng saponins wherein the method is **characterized by** the steps of :
(1) diluting iso-osmotic solution comprising sodium chloride, glucose or sorbitol with distilled water to a concentration of 80-300mg/ml of said sodium chloride, glucose or sorbitol, and filtering the same through active carbon;
(2) stirring Radix Panax Notoginseng Saponins and dissolving it in the filtrate
(3) adding pH stabilizer to the filtrate and adjusting the pH value to 6, wherein the pH stabilizer is sodium citrate, citric acid, phosphate or acetate.
(4) diluting the resultant mixture in distilled water to a concentration of the pH stabilizer of 0.1-0.5 mg/ml and a concentration of Radix Panax Notoginseng saponins of 0.1 mg - 14.0 mg Rg1 component/ml; filtering the solution until clear, and thereafter pasteurizing and packing the resulting product as Radix Panax Notoginseng Saponins intravenous injection composition.

2. The method according to Claim 1, wherein the concentration of said Radix Panax Notoginseng Saponins 1.0-7.0mg Rg1 component/ml.

3. The method according to Claim 2, wherein the concentration of said Radix Panax Notoginseng Saponins is 1.0-3.5mg Rg1 component/ml.

4. The method according to Claim 3, wherein the concentration of said Radix Panax Notoginseng Saponins is 1.4mg Rg1 component/ml.

5. The method according to Claim 1, wherein said iso-osmotic solution is sodium chloride, glucose, and sorbitol.

6. The method according to Claim 1, 2, 3, or 4 wherein said iso-osmotic solution is sodium chloride.

7. The method according to Claim 6, wherein the concentration of said sodium chloride is 7.5-9.5 mg/ml.

8. The method according to Claim 7, wherein the concentration of said sodium chloride is 8.5 mg/mL

9. The method according to any one of Claims 1 -4, wherein said pH stabilizer is sodium citrate.

10. The method according to Claim 9, wherein the concentration of said sodium citrate is 0.1-0.5 mg/ml.

11. The method according to Claim 10, wherein the concentration of said sodium citrate is 0.3 mg/ml.

12. A composition for intravenous injection comprising Radix Panax Notoginseng Saponins obtainable by the method of any one of the preceding claims.

## Patentansprüche

1. Verfahren zum Herstellen einer Zusammensetzung zur intravenösen Injektion, welche Saponine von Radix Panax Notoginseng aufweist, wobei das Verfahren **gekennzeichnet ist durch** die Schritte:
1) Verdünnen einer Natriumchlorid, Glucose oder Sorbit aufweisenden isotonischen Lösung mit destilliertem Wasser bis zu einer Konzentration von 80 ... 300 mg/ml des Natriumchlorids, der Glucose oder des Sorbits, sowie Filtrieren das ganzen **durch** Aktivkohle;
2) Rühren von Saponinen der Radix Panax Notoginseng und diese in dem Filtrat auflösen;
3) Zugeben eines Mittels zum Stabilisieren des pH-Werts zu dem Filtrat und Einstellen des pH-Werts auf 6, wobei das Mittel zum Stabilisieren des pH-Werts Natriumcitrat ist, Citronensäure, -phosphat oder -acetat ist;
4) Verdünnen der resultierenden Mischung in destilliertem Wasser bis zu einer Konzentration des Mittels zum Stabilisieren des pH-Werts von 0,1 ... 0,5 mg/ml und einer Konzentration der Saponine von Radix Panax Notoginseng 0,1 ... 14,0 mg Rg1 Komponente/ml; Filtrieren der Lösung bis zur Klarheit und danach Pasteurisieren und Verpacken des resultierenden Produkts als Zusammensetzung von Radix Panax Notoginseng Saponinen zur intravenösen Injektion.

2. Verfahren nach Anspruch 1, wobei die Konzentration der Radix Panax Notoginseng Saponine 1,0 ... 7,0 mg Rg1 Komponente / ml beträgt.

3. Verfahren nach Anspruch 2, wobei die Konzentration Radix Panax Notoginseng Saponine 1,0 ... 3,5 mg Rg1 Komponente/ml beträgt.

4. Verfahren nach Anspruch 3, wobei die Konzentration der Radix Panax Notoginseng Saponine 1,4 mg Rg1 Komponente/ml beträgt.

5. Verfahren nach Anspruch 1, wobei die isotonische Lösung Natriumchlorid, Glucose und Sorbit ist.

6. Verfahren nach Anspruch 1, 2, 3 oder 4, wobei die isotonische Lösung Natriumchlorid ist.

7. Verfahren nach Anspruch 6, wobei die Konzentration des Natriumchlorids 7,5 ... 9,5 mg/ml beträgt.

8. Verfahren nach Anspruch 7, wobei die Konzentration des Natriumchlorids 8,5 mg/ml beträgt.

9. Verfahren nach einem der vorgenannten Ansprüche 1 bis 4, wobei das Mittel zum Stabilisieren des pH-Werts Natriumcitrat ist.

10. Verfahren nach Anspruch 9, wobei die Konzentration des Natriumcitrats 0,1 ... 0,5 mg/ml beträgt.

11. Verfahren nach Anspruch 10, wobei die Konzentration des Natriumcitrats 0,3 mg/ml beträgt.

12. Zusammensetzung zur intravenösen Injektion, welche Saponine von Radix Panax Notoginseng aufweist und mit Hilfe des Verfahren nach einem der vorgenannten Ansprüche erhalten werden kann.

## Revendications

1. Procédé de préparation d'une composition pour injection intraveineuse comprenant des saponines de Radix Panax Notoginseng, dans lequel le procédé est **caractérisé par** les étapes consistant à :
(1) diluer une solution iso-osmotique comprenant du chlorure de sodium, du glucose ou du sorbitol avec de l'eau distillée jusqu'à une concentration de 80 à 300 mg/ml dudit chlorure de sodium, glucose ou sorbitol, et filtrer celle-ci à travers du charbon actif ;
(2) agiter des saponines de Radix Panax Notoginseng et les dissoudre dans le filtrat ;
(3) ajouter un stabilisateur de pH au filtrat et régler le pH à 6, dans lequel le stabilisateur de pH est le citrate de sodium, l'acide citrique, un phosphate ou un acétate ;
(4) diluer le mélange résultant dans de l'eau distillée jusqu'à une concentration du stabilisateur de pH de 0,1 à 0,5 mg/ml et une concentration des saponines de Radix Panax Notoginseng de 0,1 mg à 14,0 mg de composant Rg1/ml ; filtrer la solution jusqu'à transparence, puis pasteuriser et emballer le produit résultant en tant que composition pour injection intraveineuse de saponines de Radix Panax Notoginseng.

2. Procédé selon la revendication 1, dans lequel la concentration desdites saponines de Radix Panax Notoginseng est de 1,0 à 7,0 mg de composant Rg1/ml.

3. Procédé selon la revendication 2, dans lequel la concentration desdites saponines de Radix Panax Notoginseng est de 1,0 à 3,5 mg de composant Rg1/ml.

4. Procédé selon la revendication 3, dans lequel la concentration desdites saponines de Radix Panax Notoginseng est de 1,4 mg de composant Rg1/ml.

5. Procédé selon la revendication 1, dans lequel ladite solution iso-osmotique est du chlorure de sodium, du glucose et du sorbitol.

6. Procédé selon la revendication 1, 2, 3 ou 4, dans lequel ladite solution iso-osmotique est du chlorure de sodium.

7. Procédé selon la revendication 6, dans lequel la concentration dudit chlorure de sodium est de 7,5 à 9,5 mg/ml.

8. Procédé selon la revendication 7, dans lequel la concentration dudit chlorure de sodium est de 8,5 mg/ml.

9. Procédé selon l'une quelconque des revendications 1 à 4, dans lequel ledit stabilisateur de pH est le citrate de sodium.

10. Procédé selon la revendication 9, dans lequel la concentration dudit citrate de sodium est de 0,1 à 0,5 mg/ml.

11. Procédé selon la revendication 10, dans lequel la concentration dudit citrate de sodium est de 0,3 mg/ml.

12. Composition pour injection intraveineuse comprenant des saponines de Radix Panax Notoginseng pouvant être obtenue par le procédé selon l'une quelconque des revendications précédentes.
